# EUROPEAN PATENT APPLICATION

(11) **EP 1 707 165 A1**
(43) Date of publication of application: **04.10.2006**
(21) Application number: 05108456.4
(22) Date of filing: 15.09.2005
(51) Int. Cl.: A61F 7/02

(54) **Sheet-like face pack and kits for face packs**

(30) Priority: 28.03.2005 JP 2005091242; 13.07.2005 JP 2005204234
(71) Applicant: Ohshin MLP Co., Ltd, Takefu-shi, Fukui 915-0052 (JP)
(72) Inventor: Ota, Keizo, 915-0841, Fukui (JP); Watanabe, Tetsuhiro, 915-0841, Fukui (JP)
(74) Representative: Weihs, Bruno Konrad

(57) **Abstract**

A sheet-like face pack and kits for face packs are provided. The pack comprises a pouched part having an air-permeable side and an air-impermeable side, a substrate layer for gel that is set on the outer surface of the air-impermeable side, a gel layer that is set on the substrate layer, and an exothermic composition that is put into the pouched part, wherein a moisture permeability of the air-permeable side is within the range of 300 to 2,300 g/m². 24hours by the Lyssy method, and wherein the temperature of the gel layer reaches 38 °C within 10 minutes after the exothermic composition starts to contact oxygen and is then maintained between 38 °C and 45 °C for 20 minutes or more, wherein the temperature of the gel layer is determined according to the method for determining a temperature based on JIS S 4100.

## Description

### FIELD OF INVENTION

The present invention relates to a sheet-like face pack and kits for face packs.

### BACKGROUND

A face pack, in other words, a cosmetic pack or a facial mask, is used for making components that are effective to maintain skin healthy, e.g., moisture components, absorb into and hold in the skin and for removing excess sebum.

It has been thought that warming is effective to promote the absorption of the components. Thus, for the case where the face pack is used for, especially, making the components absorb into and hold in skin, various methods for applying the face pack to the skin under a warmed condition have been proposed.

Japanese patent early-publication No. 2004-2223 discloses a face pack consisting of an exothermic composition. The temperature of the exothermic composition increases by the heat that is generated by the hydration of zeolite.

Also, Japanese patent early-publication No. 2003-81765 discloses a pack composition comprising an exothermic component. The pack composition generates heat by itself.

Japanese patent early-publication No. Hei. 5-9109 discloses an exothermic pack composition. The exothermic component that is used in the exothermic pack composition is covered with a coating agent that is semisolid or solid under ordinary temperatures. Because the exothermic component is covered with the coating agent, the temperature of the exothermic pack composition can be controlled to be a comfortable temperature.

Japanese patent early-publication No. 2003-321342 discloses a sheet-like face pack in which a layer for accumulating heat is formed on a substrate sheet that is impregnated with a cosmetic solution. The layer comprises a polyhydric alcohol or water and a thickener or a gelling agent, and is used after it has been warmed in a hot water or by using a microwave oven.

Japanese patent early-publication No. 2002-322027 discloses a sheet-like face pack comprising a poly(norbornene) sheet containing cosmetic components and a means for controlling a temperature. Japanese patent early-publication No. 2002-322028 discloses a sheet-like face pack comprising a poly(norbornene) support and a means for controlling a temperature. Examples of the means include a steamed towel, an infrared lamp, steam, and a composition for controlling the temperature of the face pack. One example of the composition comprises, as essential components, A) a solution of an agent for solidifying alginic acid, B) a composition comprising alginic acid and/or a water-soluble alginate and a metal oxide, and C) an acid solution. In those sheet-like face packs, the poly(norbornene) sheet and the poly(norbornene) support become soft when they are warmed and therefore the packs can be readily adhered to skin. Then, the temperatures of the packs lower and the sheet and support return to sheet-like forms. Thus, the packs can be readily peeled from the surface of the skin.

Japanese patent early-publication No. Hei. 11-347059 discloses an exothermic pack for nose. In this pack an exothermic composition is put into pouched parts that are made of a non-woven fabric. It also discloses that a support that is impregnated with any pharmaceuticals may be put into each pouched part or may be attached onto the outer surface of the pack.

Japanese patent early-publication No. 2003-164501 discloses a molding for caring body into which an exothermic composition is put. The molding is made of a non-woven fabric and has projection parts which are impregnated with a liquid cosmetic.

Formulations of chemical exothermic compositions, which are used in articles that are applied to human body, are disclosed in various patent documents and the like. International publication No. WO98/29067 discloses, as one example of those formulations, a formulation of a chemical exothermic composition that is filled in heat cells of a disposable thermal neck wrap. Specifically, the chemical exothermic composition comprises 30 to 80% by weight of iron powder, 3 to 25% by weight of a carbonic material such as active or activated carbon, 0.5 to 10% by weight of a metal salt, and 1 to 40% by weight of water.

Under the above circumstances, the present inventors have extensively studied to provide a sheet-like face pack comprising a layer of an exothermic composition wherein by bringing the exothermic composition into contact with oxygen in air the exothermic composition comes to have, in a short period of time, a temperature that is appropriate for applying the face pack to skin and the temperature is maintained during the face pack is used.

Also, the present inventors have extensively studied to provide kits for face packs each comprising (a) an exothermic structure that comprises a pouched part and an exothermic composition that is put into the pouched part and that has a layered form, and (b) gel, wherein the gel is applied on the outer surface of the exothermic strucutre and then the face pack thus prepared is used.

### SUMMARY OF INVENTION

As a result of the studies, present inventors have attained to the present invention.

Namely, the present invention relates to a sheet-like face pack comprising a pouched part having an air-permeable side and an air-impermeable side, a substrate layer for gel that is set on the outer surface of the air-impermeable side, a gel layer that is set on the substrate layer, and an exothermic composition that is put into the pouched part, wherein a moisture permeability of the air-permeable side is within the range of 300 to 2,300 g/m²·24hours by the Lyssy method (JIS K 7129, at 40 degrees by Celsius temperature scale (°C) and relative humidity (RH) of 90%), and wherein the temperature of the gel layer reaches 38 degrees by Celsius temperature scale (°C) within 10 minutes after the exothermic composition starts to contact oxygen and is then maintained between 38 °C and 45 °C for 20 minutes or more, wherein the temperature of the gel layer is determined according to the method for determining a temperature based on JIS S 4100.

The sheet-like face pack of the present invention is preferably one comprising a pouched part having an air-permeable side and an air-impermeable side, a substrate layer for gel that is set on the outer surface of the air-impermeable side, a gel layer that is set on the substrate layer, and an exothermic composition that is put into the pouched part, wherein a moisture permeability of the air-permeable side is within the range of 300 to 2,300 g/m²·24hours by the Lyssy method (JIS K 7129, at 40 degrees by Celsius temperature scale (°C) and relative humidity (RH) of 90%), and wherein the temperature of the gel layer reaches 39 degrees by Celsius temperature scale (°C) within 10 minutes after the exothermic composition starts to contact oxygen and is then maintained between 39 °C and 45 °C for 20 minutes or more, wherein the temperature of the gel layer is determined according to the method for determining a temperature based on JIS S 4100.

The sheet-like face pack of the present invention includes embodiments that also have at least one of the following items (A) to (F):
(A) the substrate layer for gel is made of a non-woven fabric, a woven fabric, paper, or knit;
(B) the air-permeable side is a composite that comprises an outer layer of a material for retaining moisture and an inner layer that is made of an air-permeable polymer sheet wherein the outer layer partly adheres to the inner layer, and the air-impermeable side is made of an air-impermeable polymer sheet;
(C) there are two or more pouched parts into which the exothermic composition is put;
(D) the exothermic composition comprises, based on the total weight of the composition, 35.0 to 55.0% by weight of a metallic powder, 1.0 to 10.0% by weight (preferably 2.0 to 10.0% by weight) of a reaction auxiliary, 1.0 to 10.0% by weight of a carbon powder, and 20.0 to 40.0% by weight of water;
(E) the gel layer has been prepared from a composition comprising, based on the total weight of the composition, 5 to 15% by weight of a water-soluble polymeric compound, 0.05 to 1.00% by weight of a cross linking agent, 10 to 30% by weight of a polyhydric alcohol, and 40 to 80% by weight of water; and
(F) the gel layer has been prepared from a composition comprising, based on the total weight of the composition, 5 to 15% by weight of a water-soluble polymeric compound, 0.05 to 1.00% by weight of a cross linking agent, 10 to 30% by weight of a polyhydric alcohol, 0.01 to 10.00% by weight of a component that is effective to make or maintain skin healthy, and 40 to 80% by weight of water.

In the above item (F), the component is preferably at least one member selected from the group consisting of a placenta extract, collagen, hyaluronic acid, coenzyme Q10, amino acids, vitamins, stearic acid, olive oil, carrot oil, essential oils, vegetable extracts, seaweed extracts, and fruit extracts.

Also, the present invention relates to a kit (I) for a face pack comprising an exothermic structure (I) that comprises a pouched part having an air-permeable side and an air-impermeable side, a substrate layer for gel that is set on the outer surface of the air-impermeable side, and an exothermic composition that is put into in the pouched part, and gel that is separate from the exothermic structure (I), wherein a moisture permeability of the air-permeable side is within the range of 300 to 2,300 g/m²·24hours by the Lyssy method (JIS K 7129, at 40 degrees by Celsius temperature scale (°C) and relative humidity (RH) of 90%), and wherein within 10 minutes after preparing a gel layer by using the gel on the outer surface of the substrate layer, the temperature of the gel layer reaches 38 degrees by Celsius temperature scale (°C) and is then maintained between 38°C and 45 °C for 20 minutes or more, wherein the temperature of the gel layer is determined according to the method for determining a temperature based on JIS S 4100.

The kit (I) of the present invention is preferably one comprising an exothermic structure (I) that comprises a pouched part having an air-permeable side and an air-impermeable side, a substrate layer for gel that is set on the outer surface of the air-impermeable side, and an exothermic composition that is put into in the pouched part, and gel that is separate from the exothermic structure (I), wherein a moisture permeability of the air-permeable side is within the range of 300 to 2,300 g/m². 24hours by the Lyssy method (JIS K 7129, at 40 degrees by Celsius temperature scale (°C) and relative humidity (RH) of 90%), and wherein within 10 minutes after preparing a gel layer by using the gel on the outer surface of the substrate layer, the temperature of the gel layer reaches 39 degrees by Celsius temperature scale (°C) and is then maintained between 39 °C and 45 °C for 20 minutes or more, wherein the temperature of the gel layer is determined according to the method for determining a temperature based on JIS S 4100.

The kit (I) of the present invention includes embodiments that also have at least one of the following items (a) to (h):
(a) the substrate layer for gel is made of a non-woven fabric, a woven fabric, paper, or knit;
(b) the exothermic structure is put into an air-impermeable bag and the gel has a sheet-like form and is put into a waterproof bag;
(c) the gel has been prepared from a composition comprising, based on the total weight of the composition, 5 to 15% by weight of a water-soluble polymeric compound, 0.05 to 1.00% by weight of a cross linking agent, 10 to 30% by weight of a polyhydric alcohol, and 40 to 80% by weight of water;
(d) the exothermic structure is put into an air-impermeable bag and the gel is put into a tube or a waterproof pouch;
(e) the gel has been prepared from a composition comprising, based on the total weight of the composition, 5 to 15% by weight of a water-soluble polymeric compound, 10 to 30% by weight of a polyhydric alcohol, and 40 to 80% by weight of water;
(f) the air-permeable side is a composite that comprises an outer layer of a material for retaining moisture and an inner layer that is made of an air-permeable polymer sheet wherein the outer layer partly adheres to the inner layer, and the air-impermeable side is made of an air-impermeable polymer sheet;
(g) there are two or more pouched parts into which the exothermic composition is put; and
(h) the exothermic composition comprises, based on the total weight of the composition, 35.0 to 55.0% by weight of a metallic powder, 1.0 to 10.0% by weight (preferably 2.0 to 10.0% by weight) of a reaction auxiliary, 1.0 to 10.0% by weight of a carbon powder, and 20.0 to 40.0% by weight of water.

Further, the present invention relates to a kit (II) for a face pack comprising an exothermic structure (II) that comprises a pouched part having an air-permeable side and an air-impermeable side and an exothermic composition that is put into the pouched part, and gel that is separate from the exothermic structure (II), wherein a moisture permeability of the air-permeable side is within the range of 300 to 2,300 g/m² · 24hours by the Lyssy method (JIS K 7129, at 40 degrees by Celsius temperature scale (°C) and relative humidity (RH) of 90%), and wherein within 10 minutes after preparing a gel layer by using the gel on the outer surface of the air-impermeable side, the temperature of the gel layer reaches 38 degrees by Celsius temperature scale (°C) and is then maintained between 38 °C and 45 °C for 20 minutes or more, wherein the temperature of the gel layer is determined according to the method for determining a temperature based on JIS S 4100.

The kit (II) of the present invention is preferably one comprising an exothermic structure (II) that comprises a pouched part having an air-permeable side and an air-impermeable side and an exothermic composition that is put into the pouched part, and gel that is separate from the exothermic structure (II), wherein a moisture permeability of the air-permeable side is within the range of 300 to 2,300 g/m²·24hours by the Lyssy method (JIS K 7129, at 40 degrees by Celsius temperature scale (°C) and relative humidity (RH) of 90%), and wherein within 10 minutes after preparing a gel layer by using the gel on the outer surface of the air-impermeable side, the temperature of the gel layer reaches 39 degrees by Celsius temperature scale (°C) and is then maintained between 39 °C and 45 °C for 20 minutes or more, wherein the temperature of the gel layer is determined according to the method for determining a temperature based on JIS S 4100.

The kit (II) includes embodiments that also have at least one member selected from the above items (d) to (h) for the kit (I).

In the above item (e), the composition may further comprise, based on the total weight of the composition, 0.5 to 10.0% by weight of an inorganic pigment.

In the above items (c) and (e), the composition may further comprise, based on the total weight of the composition, 0.001 to 10.000% by weight of a fragrance.

In the above items (c) and (e), the composition may further comprise, based on the total weight of the composition, 0.01 to 10.00% by weight of a component that is effective to make or maintain skin healthy.

The component that is effective to make or maintain skin healthy is preferably at least one member selected from the group consisting of a placenta extract, collagen, hyaluronic acid, coenzyme Q10, amino acids, vitamins, stearic acid, olive oil, carrot oil, essential oils, vegetable extracts, seaweed extracts, and fruit extracts.

### BRIEF DESCRIPTION OF DRAWINGS

Figure 1 is a schematic, sectional view of an example of the sheet-like face pack according to the present invention.
Figure 2 is a schematic, sectional view of another example of the sheet-like face pack according to the present invention.
Figure 3 is a schematic, sectional view of an example of the exothermic structure (I) in the kit (I) according to the present invention.
Figure 4 is a schematic, sectional view of another example of the exothermic structure (I) in the kit (I) according to the present invention.
Figure 5 is a schematic, sectional view of an example of the exothermic structure (II) in the kit (II) according to the present invention.
Figure 6 is a schematic, sectional view of an example of the form of the gel in the kit (I) according to the present invention.
Figure 7 is a schematic view of an example of the tube in the kit (I) or (II) of the present invention wherein the tube contains the gel.
Figure 8 is a schematic view of an example of the waterproof pouch in the kit (I) or (II) of the present invention wherein the pouch contains the gel.
Figure 9 is a schematic, plane view of an example of the sheet-like face pack according to the present invention that has four pouched parts.

### DETAILED DESCRIPTION OF INVENTION

Hereafter, the present invention will be specifically explained with referring to preferable examples, to which the present invention is not limited.

First, the physical structures of the sheet-like face pack and the kits (I) and (II) according to the present invention will be explained with referring to drawings in which examples of the face pack are drawn.

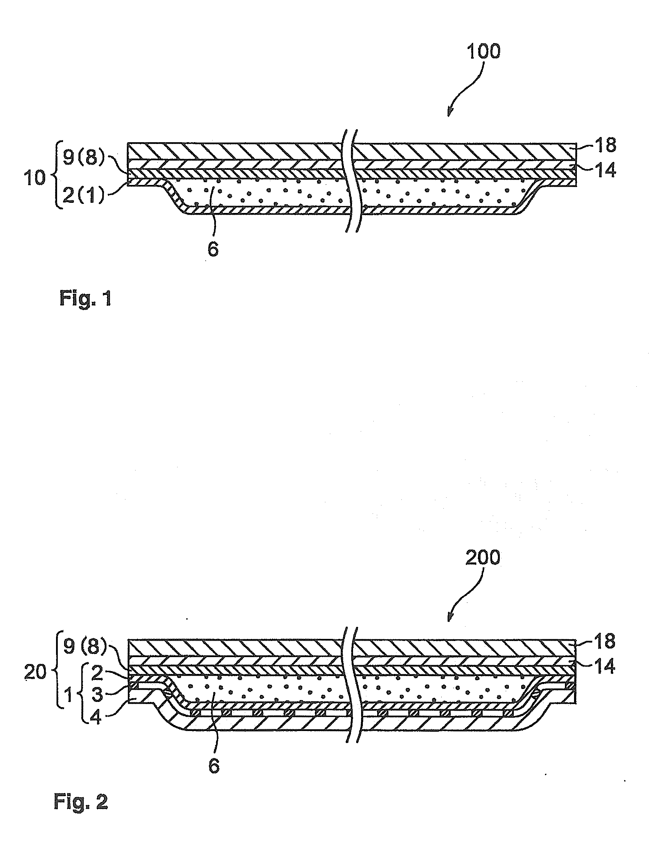

Figure 1 is a schematic, sectional view of a preferable example of the sheet-like face pack according to the present invention. The sheet-like face pack 100 comprises a pouched part 10 having an air-permeable side 1 consisting of an air-permeable polymer sheet 2 and an air-impermeable side 8 consisting of an air-impermeable polymer sheet 9, an exothermic composition 6 that is put into the pouched part 10, a substrate layer 14 for gel that is made of a non-woven fabric and is set on the outer surface of the air-impermeable side 8 of the pouched part 10, and a gel layer 18 that is set on the substrate layer 14 for gel.

Figure 2 is a schematic, sectional view of another preferable example of the sheet-like face pack according to the present invention. The sheet-like face pack 200 comprises a pouched part 20 having an air-permeable side 1 that is made of a composite comprising an air-permeable polymer sheet 2 and a non-woven fabric 4 wherein the air-permeable polymer sheet 2 partly adheres to the non-woven fabric 4 with an adhesive 3 and an air-impermeable side 8 consisting of an air-impermeable polymer sheet 9, an exothermic composition 6 that is put into the pouched part 20, a substrate layer 14 for gel that is made of a non-woven fabric and is set on the outer surface of the air-impermeable side 8 of the pouched part 20, and a gel layer 18 that is set on the substrate layer 14 for gel.

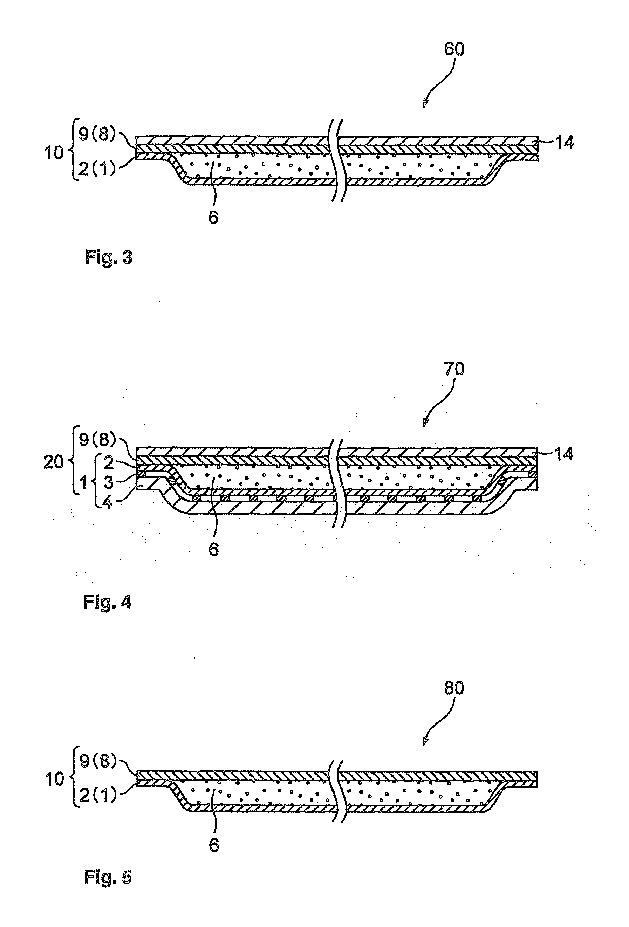

Figure 3 is a schematic, sectional view of a preferable example of the exothermic structure (I) in the kit (I) according to the present invention. The exothermic structure 60 comprises a pouched part 10 having an air-permeable side 1 that consists of an air-permeable polymer sheet 2 and an air-impermeable side 8 that consists of an air-impermeable polymer sheet 9, an exothermic composition 6 that is put into the pouched part 10, and a substrate layer 14 for gel that is made of a non-woven fabric and is set on the outer surface of the air-impermeable side 8 of the pouched part 10.

Figure 4 is a schematic, sectional view of another preferable example of the exothermic structure (I) in the kit (I) according to the present invention. The exothermic structure 70 comprises a pouched part 20 having an air-permeable side 1 that is made of a composite comprising an air-permeable polymer sheet 2 and a non-woven fabric 4 wherein the air-permeable polymer sheet 2 partly adheres to the non-woven fabric 4 with an adhesive 3 and an air-impermeable side 8 that consists of an air-impermeable polymer sheet 9, an exothermic composition 6 that is put into the pouched part 20, and a substrate layer 14 for gel that is made of a non-woven fabric and is set on the outer surface of the air-impermeable side 8 of the pouched part 20.

Figure 5 is a schematic, sectional view of a preferable example of the exothermic structure (II) in the kit (II) according to the present invention. The exothermic structure 80 comprises a pouched part 10 having an air-permeable side 1 that consists of an air-permeable polymer sheet 2 and an air-impermeable side 8 that consists of an air-impermeable polymer sheet 9, and an exothermic composition 6 that is put into the pouched part 10.

In the examples that are represented in Figures 1 to 5, the periphery of the air-permeable polymer sheet 2 is adhered to that of the air-impermeable polymer sheet 9 by heat seal. They may be adhered with an adhesive. In the case, there is also an adhesive layer between the air-permeable polymer sheet 2 and the air-impermeable polymer sheet 9 in the periphery of the pouched part 10 or 20.

In the examples that are represented in Figures 2 and 4, the air-permeable polymer sheet 2 partly adheres to the non-woven fabric 4 with an adhesive 3 so as to maintain air permeability. Instead of the use of the adhesive 3, the air-permeable polymer sheet 2 may be partly fused the non-woven fabric 4. Or, there may be an embodiment in which the periphery of the air-permeable polymer sheet 2 is adhered to or fused that of the non-woven fabric 4 and their inner parts (i.e., the parts of air-permeable polymer sheet 2 and the non-woven fabric 4 that correspond to the portion where the exothermic composition 6 contacts the air-permeable polymer sheet 2) are not adhered or fused each other.

In the examples that are represented in Figures 1 to 4, the entire or periphery of the air-impermeable polymer sheet 9 is heat-sealed or adhered with an adhesive to that of the substrate layer 14 for gel that is made of a non-woven fabric.

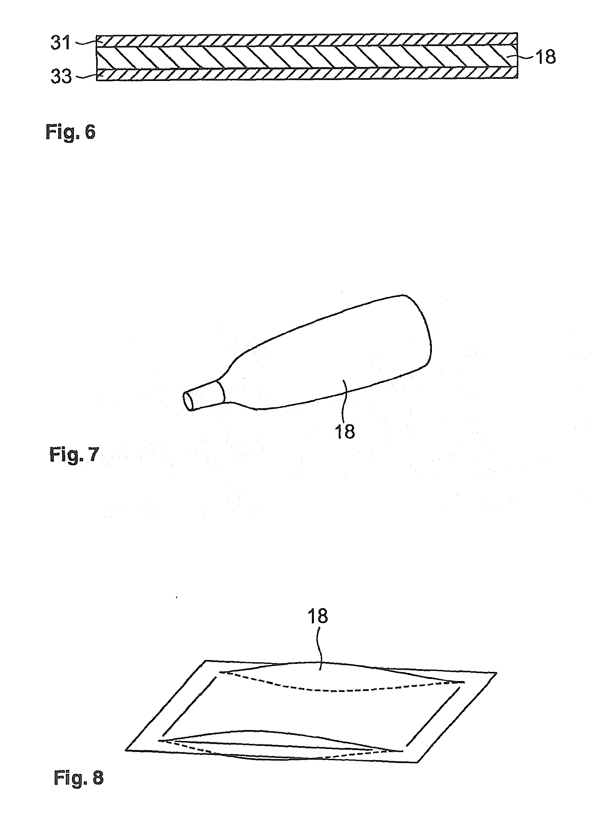

Figure 6 is a schematic, sectional view of an example of the form of the gel in the kit (I) according to the present invention. In this example, the gel 18 has a sheet-like form and is put between two protective sheets 31, 33.

Figure 7 is a schematic view of a tube C in the kit (I) or (II) of the present invention wherein the tube C holds the gel 18.

Figure 8 is a schematic view of a waterproof pouch D in the kit (I) or (II) of the present invention wherein the pouch D holds the gel.

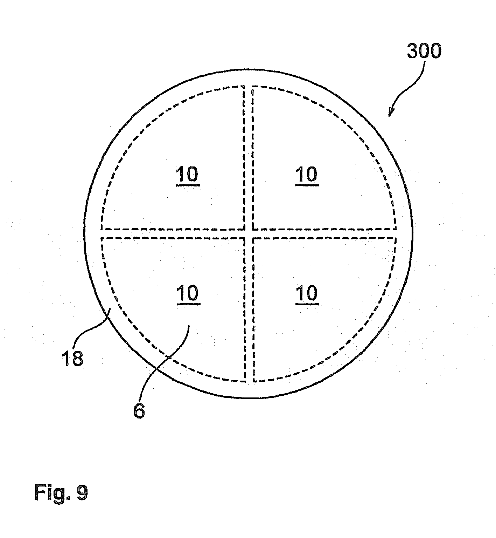

Figure 9 is a schematic, plane view of an example of the sheet-like face pack 300 according to the present invention that has four pouched parts 10, 10, 10, 10. In this example, there is a gel layer 18 all over the substrate layer 14 for gel of the sheet-like face pack 300. An exothermic composition 6 is put into each pouched part.

Appropriate portions of the sheet-like face pack 300, e.g., portions that correspond to eyes, nasal cavity, or mouth, may be cut away. Peripheral heat-sealed portions may have some nicks. Or, the form may be oval. These embodiments may better fit the three-dimensional form of face.

Hereafter, materials and the like will be explained that are used in the sheet-like face pack and the kits (I) and (II) according to the present inventions.

The pouched part into which the exothermic composition is put has an air-permeable side and an air-impermeable side. Namely, its one side is air-permeable and the other side is air-impermeable. Usually, the air-permeable side comprises an air-permeable polymer sheet only or an air-permeable polymer sheet at the inner side and a layer of a material for retaining moisture at the outer side. The air-impermeable side preferably consists of an air-impermeable polymer sheet.

Examples of polymers as materials of the air-permeable polymer sheet and the air-impermeable polymer sheet that constitute the pouched part include polyolefins such as polyethylene and polypropylene; polyamides such as nylons; polyesters such as polyethylene terephthalate; ethylene copolymers such as an ethylene-vinyl acetate copolymer and its saponified ones and an ethylene-alkyl (meth)acrylate coplymer; poly(vinyl chloride); poly(vinylidene chloride); polyurethane; polystyrene; and polycarbonate. Natural rubber, reclaimed rubber, and synthetic rubber may also be used.

A representative example of the air-permeable polymer sheet is one that has been made by making openings or vents in an air-impermeable polymer sheet, e.g., a moisture-permeable porous film. In this description and claims, the terms "moisture-permeable" and "moisture permeability" may be used. If moisture can pass through, gas such as air can also pass through. Namely, one having a moisture permeability has a gas or air permeability.

If the moisture-permeable porous film is used as the air-permeable polymer sheet, its thickness is usually 100 *µ* m (micrometer) or less, preferably 20 to 80 *µ* m, and more preferably 40 to 60 *µ* m.

An example of the air-impermeable polymer sheet that constitutes the other side of the pouched part is an air-impermeable polyethylene film. The thickness of the air-impermeable polymer sheet is usually 100 *µ* m (micrometer) or less, preferably 10 to 70 *µ* m, more preferably 20 to 60 *µ* m, and especially preferably 30 to 50 *µ* m.

The air-permeable polymer sheet and the air-impermeable polymer sheet are not limited to monolayer films and may be multilayer films.

It is preferable that at least one of the inmost layer of the air-permeable side and the inmost layer of the air-impermeable side is a polymer film having a heat sealability, e.g., a polyethylene film that has been polymerized by using a matallocene as the catalyst.

The air-permeable side may be constituted of only an air-permeable polymer sheet. Alternatively, the side may be constituted of an air-permeable polymer sheet and a layer of a material for retaining moisture. In the latter case, as shown in Figures 2 and 4, the layer of the material for retaining moisture, e.g., a non-woven fabric 4, is outside the air-permeable polymer sheet 2. Here, the layer of the material for retaining moisture has to be air-permeable. Examples of materials that constitute the layer of the material for retaining moisture include woven fabrics, non-woven fabrics, knits, and paper.

If the layer of the material for retaining moisture is made of a woven fabric, a non-woven fabric, or paper, the thickness of the layer is, as represented by a basis weight, usually 200 g/m² or less, preferably 30 to 120 g/m², and more preferably 40 to 100 g/m². The non-woven fabric is preferably spun lace or spun bond one. Examples of raw materials of the non-woven fabric include rayon, nylons, polyesters, acrylics, polypropylene, vinylon, polyethylene, polyurethanes, cotton, and cellulose.

The air permeability of the air-permeable side affects the exothermic property of the exothermic composition. Thus, in the present invention a material or materials that constitute(s) the air-permeable side is(are) selected and processed so that the air-permeable side has a moisture permeability of 300 to 2,300 g/m². 24 hours, preferably 400 to 1,500 g/m². 24 hours, and more preferably 500 to 1,000 g/m². 24 hours by the Lyssy method (JIS K 7129 (1992), at 40 degrees by Celsius temperature scale (°C) and relative humidity (RH) of 90%).

The Lyssy method is compliant to industry standards of many countries. In, e.g., JIS Z 0208, JIS K 7129, and ASTM E 398, the measurement by the Lyssy method is conducted at 40 degrees by Celsius temperature scale under relative humidity, i.e., the difference of humidity, of 90%. More particularly, a sample to be measured is inserted into the interface of an underneath chamber that lies under a condition of relative humidity of 100% and an upper chamber comprising a high-sensitive humidity sensor. The relative humidity in the upper chamber is 10%. Thus, the difference of the relative humidity is 90% (i.e., 100% - 10%). Then, the time (seconds) to be required for increasing the relative humidity of the upper chamber from about 9% to about 11% is measured. By using a standard sample, of which the moisture permeability is known, the time is measured in the same way under the same conditions. Comparing the data of the sample to be measured with that of the standard sample, the moisture permeability of the sample to be measured is decided.

The moisture permeability is determined by using a material that is to be the air-permeable side of the pouched part.

When the air-permeable side is constituted of plural layers, the moisture permeability is determined by making air pass from the outermost layer (i.e., a layer that contacts air) to the inmost layer (i.e., a layer that contacts the exothermic composition) of the air-permeable side.

Methods for processing a material or materials that constitute(s) the air-permeable side so that the air-permeable side has a desirable moisture permeability have been known in this technical field. More specifically, methods for controlling the moisture permeability by, as shown in Figures 2 and 4, partly adhering the air-permeable polymer sheet 2 with the non-woven fabric 4 and a method for preparing a porous film having a desired moisture permeability have been known.

The exothermic composition that is used in the present invention generates heat in the presence of oxygen. The components that are contained in the exothermic composition are not limited as long as they have been used in the conventional exothermic compositions. Examples of the components are as follows:

Examples of chemical exothermic agents include metal powders such as iron powders, especially reduced iron powder and atomized iron powder. Examples of reaction auxiliaries include metal halides such as sodium chloride, potassium chloride, magnesium chloride, calcium chloride, iron (II) chloride, and iron (III) chloride; and metal sulfates such as potassium sulfate, sodium sulfate, magnesium sulfate, copper (II) sulfate, iron (II) sulfate, and iron (III) sulfate. As the auxiliaries for generating heat, carbon powders such as active (or activated) carbon powders are usually used. Examples of the active carbon include those derived from carbon of coconut shell, grounds obtained by extracting coffee, charcoal powder, coal, bituminous coal, and peat coal. The exothermic composition also contains water. Further, examples of water retaining agents include alumina, silica gel, zeolite, and water-absorptive polymeric compounds such as carboxymethyl cellulose and acrylic acid starch. Examples of other additives include polymeric compounds such as polyethylene, polypropylene, and polystyrene; and fillers such as wood powder, charcoal, bentonite, vermiculite, and pearlite.

An exothermic composition comprising, based on its total weight, 35.0 to 55.0% by weight of a metallic powder, 1.0 to 10.0% by weight of a reaction auxiliary, 1.0 to 10.0% by weight of a carbon powder, and 20.0 to 40.0% by weight of water is preferably used. The amount of the reaction auxiliary is preferably 2.0 to 10.0% by weight

An exothermic composition having a formula is preferably used such that a metal powder such as iron powder is evenly located.

It is also preferable that the exothermic composition is processed to be a sheet-like form. The thickness of the sheet-like exothermic composition is preferably 5 mm or less, more preferably 0.5 to 4.0 mm, and especially preferably 1 to 2 mm.

The conventional methods for preparing the exothermic composition and the conventional methods for processing the exothermic composition to be the sheet-like form may be practiced.

The summary of an example of the method for preparing the exothermic composition is as follows:

Namely, iron powder, wood powder, and active carbon are mixed to one another. To the thus-prepared mixture, carboxymethyl cellulose and acrylic acid starch are added and the obtained mixture is stirred. Salt water is also added and then stirring is continued.

One of the characteristics of the face pack according to the present invention is that when the temperature of the gel layer is determined according to the method for determining the temperature based on JIS S 4100, the temperature of the gel layer reaches 38 degrees by Celsius temperature scale (°C) within 10 minutes (preferably within 8 minutes) after an airproof bag into which the sheet-like face pack has been put is opened, namely, after the exothermic composition starts to contact oxygen in air that reaches the exothermic composition through the air-permeable side, and is then maintained between 38°C and 45°C for 20 minutes or more (preferably 30 minutes to two hours and more preferably 40 minutes to 1.5 hours). Preferably, the temperature of the gel layer reaches 39 °C within 10 minutes (more preferably within 8 minutes) after an airproof bag into which the sheet-like face pack has been put is opened and is then maintained between 39°C and 45°C for 20 minutes or more (more preferably 30 minutes to two hours and still more preferably 40 minutes to 1.5 hours).

When the kit (I) or (II) is used, the gel layer is quickly made immediately after the exothermic structure (I) or (II) has been taken out from an airproof bag. The temperature of the gel layer reaches 38 degrees by Celsius temperature scale (°C) within 10 minutes (more preferably within 8 minutes) after the gel layer has been made and is then maintained between 38°C and 45°C (degrees by Celsius temperature scale) for 20 minutes or more (more preferably 30 minutes to two hours and still more preferably 40 minutes to 1.5 hours). Preferably, the temperature of the gel layer reaches 39 degrees by Celsius temperature scale (°C) within 10 minutes (more preferably within 8 minutes) after the gel layer has been made and is then maintained between 39°C and 45°C (degrees by Celsius temperature scale) for 20 minutes or more (more preferably 30 minutes to two hours and still more preferably 40 minutes to 1.5 hours).

The temperature of the gel layer and the duration of that temperature are mainly controlled by the moisture permeability of the air-permeable side of the pouched part and the amount and the formula of the exothermic composition.

The substrate layer for gel in the present inventions lies outside the air-impermeable side. The substrate layer for gel is entirely or partly adhered to or fused the air-impermeable side and thus united with the pouched part. On the substrate layer for gel, a gel layer is set or is to be set. The substrate layer for gel may be impregnated with part of the gel.

The material of the substrate layer for gel is not limited as long as it has an affinity for the gel. Examples of the material include a non-woven fabric, a woven fabric, paper, and a knit. Among those examples the non-woven fabric is preferable. Although the kind of the non-woven fabric is not especifically limited, spun lace or spun bond one is preferable. A non-woven fabric having a basis weight of 200 g/m² or less is preferable. The basis weight is more preferably 30 to 120 g/m² and still more preferably 40 to 100 g/m².

As the material for the non-woven fabric, a mixture of a hydrophilic fiber and a hydrophobic fiber is preferably used. This is because the hydrophilic fiber has an affinity for the gel. In the mixture, the ratio of the hydrophilic fiber is preferably 10% by weight or more.

Examples of the hydrophilic fiber include natural fibers such as cotton, wool, silk, hemp, and wood pulp; cellulose fibers such as rayon and cupra; poly(vinyl alcohol) fibers; cellulose-acetate fibers; and highly water-absorbable fibers such as crosslinked acrylate fibers, processed acrylic fibers of which surfaces are hydrolyzed, and fibers that have been obtained by graft-polymerizing acrylic acid or methacrylic acid to fibers such as polyester fibers. Examples of the hydrophobic fibers include polyester fibers, nylon fibers, and acrylic fibers.

The gel that lies on the substrate layer for gel in the sheet-like face pack according to the present invention and the gel in the kit (I) or (II) according to the present invention are prepared by using a composition for gel. The components of the composition are not specifically limited as long as they have been conventionally used for gelatinous face pack or facial mask. Examples of the components are as follows:

As water-soluble polymeric compounds, natural, semi-synthetic, and synthetic ones may be used.

Examples of the natural water-soluble polymeric compounds include guar gum (Cyamopsis Tetragonoloba Gum), locust been gum (Ceratonia Siliqua Gum), quins seed (Pyrus Cydonia Seed), carageenan, galactan, gum Arabic, tragacanth gum, pectin, mannan, starch, xanthan gum, dextran, succinoglucan, cardlan, hyaluronic acid, casein, albumin, and collagen.

Examples of the semi-synthetic water-soluble polymeric compounds include cellulose-type polymers such as methylcellulose, ethylcellulose, hydroxyethyl cellulose, hydroxypropyl cellulose, carboxymethyl cellulose, and their salts (e.g., sodium salts and potassium salts), and methyl hydroxypropyl cellulose; starch-type polymeric compounds such as soluble starch, acrylic acid starch, carboxymethyl starch, and methyl starch; alginic acid-type polymeric compounds such as propylene glycol alginates, and salts of alginic acid; and polysaccharide derivatives.

Examples of the synthetic water-soluble polymeric compounds include poly(vinyl alcohol), poly(vinyl pyrrolidone), poly(vinyl methyl ether), a carboxyvinyl polymer, poly(sodium acrylate), poly(ethylene oxide), a fatty acid ester of polyoxyethylene sorbitan, a copolymer of a long-chain alkyl (number of carbon: 10 to 30) ester of acrylic acid and that of methacrylic acid, and a block copolymer of ethylene oxide and propylene oxide.

Examples of crosslinking agents that crosslink the water-soluble polymeric compounds include edetates, calcium chloride, calcium hydroxide, calcium carbonate, calcium phosphate, calcium citrate, aluminum compounds such as aluminum chloride, potassium alum, aluminum sulfate, aluminum stearate, magnesium metasilicate aluminate, and aluminum hydroxide; and multifunctional epoxy compounds. If the gel is not sheet-like but is put into a tube or a waterproof pouch, the composition for gel contains no crosslinking agent or contains it in only a little amount.

Specific examples of polyhydric alcohols that are moisture-retaining components include ethylene glycol, diethylene glycol, triethylene glycol, glycerol, diglycerol, dynamite glycerol, propylene glycol, dipropylene glycol, 1,3-butylene glycol, 1,4-butylene glycol, sorbitol, xylitol, mannitol, multitol, galactose, glucose, fructose, maltose, lactose, polyethylene glycol, polyglycerol, polyethylene glycol monoethyl ether, diethylene glycol monoethyl ether, ethylene glycol dimethyl ether, 1,2-pentanediol, hexylene glycol, and mabit.

Examples of the component that is effective to make or maintain skin healthy, i.e., the component for beautiful skin, include a placenta extract, collagen, hyaluronic acid, coenzyme Q10, amino acids, vitamins, stearic acid, olive oil, carrot oil, essential oils, vegetable extracts, seaweed extracts, and fruit extracts. Specific examples of the amino acids include alanine and alginine. Specific examples of the vitamins include vitamins A, C, and E. A specific example of the vegetable extracts includes chamomile extract.

The composition that is used to prepare the gel contains water in addition to the above components. The composition may contain, as particle components, a colorant, a pH adjuster, an antioxidant, and a preservative. In the kits (I) and (II) according to the present invention, the gel may contain a fragrance. This is because the gel in the kits (I) and (II) does not contact the exothermic composition by the time when the gel layer is made by using the gel.

The composition for preparing the gel is not limited to a transparent or translucent one. It may be opaque because it contains at least one of titanium oxides such as titanium dioxide; inorganic pigments such as zinc oxide, iron oxides, kaolin, and bentonite; and clay.

The composition for preparing the gel preferably comprises, based on the total weight of the composition, 5 to 15% by weight of a water-soluble polymeric compound, 0.05 to 1.00% by weight of a crosslinking agent, 10 to 30% by weight of a polyhydric alcohol, and 40 to 80% by weight of water. The crosslinking agent is used in an amount of preferably 0.1 to 5 equivalents and particularly preferably 0.2 to 2 equivalents per crosslinking point of the water-soluble polymeric compound.

The composition comprises preferably 6 to 13% by weight and particularly preferably 7 to 12% by weight of the water-soluble polymeric compound. The composition comprises preferably 0.06 to 0.80% by weight and particularly preferably 0.10 to 0.50% by weight of the crosslinking agent. The composition comprises preferably 12 to 25% by weight and particularly preferably 13 to 20% by weight of the polyhydric alcohol. The composition comprises preferably 50 to 75% by weight and particularly preferably 60 to 70% by weight of the water.

The sheet-like gel may be prepared, for example, as follows:
1) among raw materials other than the crosslinking agent and a solvent for the crosslinking agent, polymers, colorants, preservatives, and the like are respectively dissolved in solvents such as water by mixing and, if necessary, heating the obtained mixtures, respectively;
2) the crosslinking agent is dissolved in the solvent by mixing and heating the obtained mixture;
3) the solutions that were obtained in the step 1 are poured into a vessel in any order while mixing;
4) the solution of the crosslinking agent is added to the vessel while mixing;
5) the obtained mixture is mixed;
6) the obtained mixture is run into a container having a predetermined form or is applied on, e.g., a protective sheet; and
7) the water-soluble polymeric compound is crosslinked by the crosslinking agent so that the mixture hardens.

The gel that is to be put into, e.g., a tube may be prepared, for example, as follows:
1) the raw materials are respectively dissolved in solvents such as water by mixing and, if necessary, heating the obtained mixtures, respectively;
2) the solutions that were obtained in the step 1 are poured into a vessel in any order while mixing; and
3) the obtained mixture is mixed.

The sheet-like gel in the present invention has hardness such that the gel can maintain a sheet-like form.

The thickness of the sheet-like gel is not limited. However, it may be 200 to 3,000 *µ*m (micrometer), is preferably 500 to 2,000 *µ*m, and is particularly preferably 800 to 1,500 *µ* m.

In the case where the gel is put into a tube or a waterproof bag, the viscosity of the gel is preferably 100 to 1,200 cps and more preferably 200 to 800 cps at 30 degrees by Celsius temperature scale (°C). If the gel has a viscosity within the above range, it can be readily applied onto an exothermic structure and is hardly ever dripped.

In the kit (I) of the present invention, the gel is provided in the form, for example, that 1) it has a sheet-like form and is directly put into a waterproof bag B, that 2) it has a sheet-like form, is put between the two protective sheets 31, 33, and is put into the waterproof bag B as shown in Figure 6, that 3) it is put into, e.g., the tube C that is represented in Figure 7, that 4) it is put into the waterproof bag D as shown in Figure 8, or that 5) it is packed by any other means, and is applied onto the substrate for the gel just before its use.

The waterproof bag B is not particularly limited as long as it is made of a waterproof and air-impermeable material and has a form and size so that the sheet-like gel can be put into the bag B as it is, i.e., in the sheet-like form. The tube C and the waterproof bag D are not particularly limited as long as they are made of a waterproof and air-impermeable material and can hold the gel while maintaining its gelatinous form.

The waterproof bags B and D are made of, for example, a multi-layered sheet of a poly(vinylidene chloride)-coated, biaxial stretched polypropylene (KOP) film and a polyethylene (PE) film, a double-layered sheet of a nylon film and a PE film, a double-layered sheet of a polyethylene terephthalate (PET) film and a PE film, a double-layered sheet of a thin-layered aluminum and a PE film, or a multi-layered sheet of paper, a thin-layered aluminum, and a PE film. The tube C may be, for example, one that is made of a synthetic polymer such as a polycarbonate, one that is made of a paint-applied, thin-layered aluminum, or a laminate one comprising five to ten layers each of which may be a polyethylene film, thin paper, or aluminum foil.

If the gel has a sheet-like form in the kit (I) of the present invention, the gel sheet is applied onto the substrate for the gel of the exothermic structure immediately after the exothermic structure (I) has been taken out from the airproof bag and just before use.

If the gel is put into a tube or the like, the gel is squeezed out and evenly applied by using fingers or a pallet onto the substrate for the gel of the exothermic structure (I) or the air-impermeable side of the exothermic structure (II) just before use.

In the kit (II) of the present invention, the gel is provided in the form, for example, that 1) it is put into, e.g., the tube C that is represented in Figure 7, that 2) it is put into the waterproof bag D as shown in Figure 8, or that 3) it is packed by any other means, and is applied on the outer surface of the air-impermeable side of the exothermic structure (II) just before use.

In the sheet-like face pack according to the present invention, before use the surface of the gel is usually covered with a releasable sheet. The material for the releasable sheet is not limited as long as it has been conventionally used as a material for the sheet that has been used to cover the surface of the gel. For example, a plastic film, a metal foil, or a composite of a plastic film and paper is used as the releasable sheet. Examples of polymeric compounds that are raw materials of the plastic film include polyesters, polypropylene, polyethylene, alkylbenzene sulfonates, and poly(vinyl chloride).

Onto the inner side (namely, a side that faces the gel) of the releasable sheet, a coating agent for release may be applied. The coating agent may be a silicone type, an alkylacrylate type, a fluorine type, or the like.

In the case where the gel has a sheet-like form and is put between two protective sheets in the kit (I) of the present invention, the material of the protective sheets is not limited as long as it has been conventionally used in the protective sheets for covering a surface of gel. Onto the two protective sheets, a coating agent for release may be applied.

The sheet-like face pack with the proviso that the surface of the gel layer is covered with a releasable sheet and the exothermic structures (I) and (II) in the kits (I) and (II) are kept in a bag as a case. The bag is made of a waterproof or moisture resistant, air-impermeable or airproof material. Because the bag is air-impermeable, the exothermic agent in the exothermic composition does not chemically react. Thus the exothermic composition is kept without generating heat. After opening the bag, air (oxygen) gets to the exothermic agent through the air-permeable side of the pouched part. Then, chemical reaction starts and heat of reaction is emitted.

A representative example of the material for the bag as the case is a composite of aluminum foil and a polymer film.

Hereafter, examples of the process for preparing the sheet-like face pack of the present invention, with the proviso that the surface of the gel layer is covered with a releasable sheet, will be explained.

Here, the process for preparing the sheet-like face pack 200 as shown in Figure 2 will be explained.
1) A composite sheet A is prepared wherein an air-impermeable polymer sheet that will construct the air-impermeable side 8 is adhered to one side of a non-woven fabric that will construct the substrate layer 14 for gel.
2) A composition for gel is applied onto the non-woven fabric of the composite sheet A, and the surface of the applied composition is covered with a releasable sheet. This is left until the composition hardens into the gel layer 18.
3) On a surface of an air-permeable polymer sheet that will construct the layer of the air-permeable polymer sheet 2, an adhesive 3 is partly applied. The layer of the adhesive 3 thus applied has preferably a form of check, polka dots, or stripes. By adjusting the ratio of the area of the adhesive 3, namely, (the total area of the plane surfaces of the parts where the adhesive 3 exists) divides (the area of the air-permeable side 1 with the proviso that the outer peripheries where no exothermic composition exists are excluded), the air permeability, i.e., the moisture permeability, of the air-permeable side 1 is appropriately adjusted.
4) A non-woven fabric is mounted on the adhesive 3 that has been applied onto the air-permeable polymer sheet. Thus, a composite sheet B that will construct the air-permeable side 1 is prepared.
5) An exothermic composition is prepared.
6) On the air-impermeable polymer sheet of the composite sheet A, an appropriate amount of the exothermic composition 6 is put so that it has a desired form.
7) A hot melt adhesive is sprayed on the exothermic composition 6 and the surrounding air-impermeable polymer sheet of the composite sheet A.
8) The exothermic composition 6 is covered with the composite sheet B wherein the air-permeable polymer sheet faces the exothermic composition 6. In the parts where there are no exothermic compositions 6, the composite sheet A is adhered to the composite sheet B with the hot melt adhesive thus sprayed.
9) One sheet-like face pack is cut off and is put into an air-impermeable or airproof bag as a case.
   Instead of the above steps 7 and 8, the following step 10 may be performed.
10) The exothermic composition 6 is covered with the composite sheet B wherein the air-permeable polymer sheet faces the exothermic composition 6. In the parts where there are no exothermic compositions 6, the composite sheet A is heat-sealed to the composite sheet B.

The process for preparing the sheet-like face pack 100 as shown in Figure 1 is the same as that for preparing the sheet-like face pack 200, except that instead of the composite sheet B, only an air-permeable polymer sheet is used as the material for the air-permeable side 1.

Hereafter, examples of the process for preparing the exothermic structure (I) in the kit (I) of the present invention will be explained.
(1) Process for Preparing Exothermic Structure 60 Shown in Figure 3
   1) A composite sheet A is prepared wherein an air-impermeable polymer sheet that will construct the air-impermeable side 8 is adhered to one side of a non-woven fabric that will construct the substrate layer 14 for gel.
   2) An exothermic composition is prepared.
   3) On the air-impermeable polymer sheet of the composite sheet A, an appropriate amount of the exothermic composition 6 is put so that it has a desired form.
   4) A hot melt adhesive is sprayed on the exothermic composition 6 and the surrounding air-impermeable polymer sheet of the composite sheet A.
   5) The exothermic composition 6 is covered with an air-permeable polymer sheet. In the parts where there are no exothermic compositions 6, the composite sheet A is adhered to the air-permeable polymer sheet with the hot melt adhesive thus sprayed.
   6) One exothermic structure 60 is cut off and is put into an air-impermeable or airproof bag as a case.
(2) Process for Preparing Exothermic Structure 70 Shown in Figure 4
   1) A composite sheet A is prepared wherein an air-impermeable polymer sheet that will construct the air-impermeable side 8 is adhered to one side of a non-woven fabric that will construct the substrate layer 14 for gel.
   2) On a surface of an air-permeable polymer sheet that will construct the layer of the air-permeable polymer sheet 2, an adhesive 3 is partly applied.
   3) A non-woven fabric is mounted on the adhesive 3 that has been applied onto the air-permeable polymer sheet. Thus, a composite sheet B that will construct the air-permeable side 1 is prepared.
   4) An exothermic composition is prepared.
   5) On the air-impermeable polymer sheet of the composite sheet A, an appropriate amount of the exothermic composition 6 is put so that it has a desired form.
   6) A hot melt adhesive is sprayed on the exothermic composition 6 and the surrounding air-impermeable polymer sheet of the composite sheet A.
   7) The exothermic composition 6 is covered with the composite sheet B wherein the air-permeable polymer sheet faces the exothermic composition 6. In the parts where there are no exothermic compositions 6, the composite sheet A is adhered to the composite sheet B with the hot melt adhesive thus sprayed.
   8) One exothermic structure 70 is cut off and is put into an air-impermeable bag as a case.

The above exothermic structures 60 and 70 may be prepared by a conventionally known process. Namely, a roll (i.e., long size one) of the air-permeable polymer sheet or the composite sheet B, which will construct the air-permeable side 1, and a roll (i.e., long size one) of the composite sheet A are run through a heat sealer to produce a pouched part, an exothermic composition is put into the thus-produced pouched part, heat sealing is performed, and one exothermic structure is cut off.

The process for preparing the exothermic structure (II) in the kit (II) is the same as that for preparing the exothermic structure (I) in the kit (I), except that instead of the composite sheet A, only an air-impermeable polymer sheet is used.

The method for using the sheet-like face pack is that the pack is taken out from the bag as the case, that the releasable sheet is peeled off, and that the gel layer is touched to a face.

The kit (I) is used, for example, as follows: First, the exothermic structure (I) is taken out from the bag as the case. The sheet-like gel is taken out from its case, e.g., a bag. The sheet-like gel is applied onto the substrate layer for the gel of the exothermic structure (I) to prepare a sheet-like face pack. If the gel does not have a sheet-like form, the gel is applied onto the substrate layer for the gel of the exothermic structure (I). The layer of the gel is touched to a face.

The kit (II) is used, for example, as follows: First, the exothermic structure (II) is taken out from the bag as the case. The gel is squeezed from, e.g., a tube and applied onto the air-impermeable side of the exothermic structure (II) to prepare a face pack. The layer of the gel is touched to a face.

After a predetermined period of time, the face pack is peeled from the face. If the gel remains on the face, the face is washed or the gel is wiped with a cosmetic lotion.

The method for determining the temperature of the gel layer based on JIS S 4100 is as follows:

### (1) Apparatus for Examination

The apparatus comprises a warming part and a thermostat vessel having a circulation system.

### (2) Warming Part

This part is made of SUS 304 (JIS G 4303) having a thickness of 3 mm. The part has a form of a box having a size of 300 mm X 600 mm X 100 mm.

On the plane of 300 mm X 600 mm, a polyacrylic plate having a size of 300 mm X 600 mm X 6 mm (thickness) is put. Other sides of the box are covered with insulators made of foam polystyrene.

### (3) Thermostat Vessel Having Circulation System

This is a vessel for circulating warm water to the warning part at a flow rate of 12 ± 1 liter per minute. The temperature of water is regulated so that the temperature of the surface of the polyacrylic plate is maintained at a temperature of 33 ± 2 degrees by Celsius temperature scale (°C).

### (4) Method for Examination

The surroundings are maintained at a temperature of 20 ± 2 degrees by Celsius temperature scale (°C). A face pack is taken out from a bag, a releasable sheet on a gel layer is peeled off, the face pack is promptly mounted on the polyacrylic plate so that the gel layer faces the polyacrylic plate, and the temperature of the gel layer is determined by using a thermal sensor.

The sheet-like face pack and the kits (I) and (II) according to the present invention exhibit an effect such that by bringing an exothermic composition into contact with oxygen in air, the exothermic composition comes to have, in a short period of time, a temperature that is appropriate for applying a face pack to skin and the temperature is maintained during the face pack is used.

When the sheet-like face pack or the kit (I) or (II) according to the present invention is used, the gel is warmed. Thus, in the present invention a higher effect can be obtained than the case where a pack is practiced under an ambient temperature from the viewpoint of making or maintaining skin healthy.

The kits (I) and (II) according to the present invention can also exhibit an effect that the gel can be fragrant, in other words, a fragrance can be added to the gel. Thus, when a pack is practiced with the fragrant gel, a relaxation effect by the fragrance can also be obtained.

### EXAMPLES

Hereafter, the present invention will be specifically explained with referring to examples.

### Example 1: Preparations and Examinations of Sheet-like Face Packs

### (1) Preparations of Exothermic Compositions

The exothermic compositions having formulas A to F shown in Table 1 were prepared. Specifically, 1) an iron powder, an active carbon and a wood powder were fully mixed to one another to prepare a mixture A; 2) a powder of carboxymethyl cellulose and acrylic acid starch were fully mixed to each other to prepare a mixture B; 3) a salt water was prepared by dissolving sodium chloride to an ordinary water; and 4) the mixture B was added to the mixture A, the obtained mixture was fully mixed, the salt water was added, and then the thus-obtained mixture was fully mixed.

### (2) Preparation of Composition for Gel

The composition for gel having the formula shown in Table 2 was prepared according to an ordinary method. Specifically, 1) poly(vinyl alcohol) was added to an ordinary water to prepare a part A; 2) a colorant and tartaric acid were added to an ordinary water to prepare a part B; 3) the part A was added to the part B to prepare a part C; 4) carboxymethyl cellulose sodium salt was added to the part C to prepare a part D; 5) acrylic acid starch was added to an amount, which corresponded to 3% by weight based on the total weight of the composition, of glycerol to prepare a part E; 6) the part E and sorbitol were added to the part D to prepare a part F; 7) butyl parahydroxybenzoate and methyl parahydroxybenzoate were added to propylene glycol to prepare a part G; 8) the part G was added to the part F to prepare a part H; 9) sorbitan (polyoxyethylene) monolaurate was added to an essential oil to prepare a part I; 10) the part I was added to the part H to prepare part J; 11) poly(sodium acrylate), a dried alminium hydroxide gel, magnesium metasilicate aluminate, and sodium edetate were added to the residual propylene glycol (an amount that corresponded to 9% by weight based on the total weight of the composition) to prepare a part K; and 12) the part K was added to the part J.

**Table 2**

| Components | Amounts (wt. %) |
|---|---|
| Carboxymethyl cellulose sodium salt | 2.30 |
| Acrylic acid starch | 1.00 |
| Tartaric acid | 0.40 |
| Propylene glycol | 0.10 |
| Butyl parahydroxybenzoate | 0.10 |
| Methyl parahydroxybenzoate | 0.10 |
| Sorbitan (polyoxyethylene) monolaurate | 0.55 |
| Poly(sodium acrylate) | 6.00 |
| Dried alminium hydroxide gel | 0.05 |
| Magnesium metasilicate aluminate | 0.07 |
| Sodium edetate (EDTA-Na) | 0.05 |
| Glycerol | 12.00 |
| Essential oil | 1.28 |
| Sorbitol | 4.00 |
| Poly(vinyl alcohol) | 2.00 |
| Colorant | particle |
| Ordinary water | balance |
| Total | 100.00 |

### (3) Determinations of Moisture Permeabilities of Air-permeable Sides

The moisture permeability of the air-permeable side of the pouched part of each sheet-like face pack was determined according to the Lyssy method (JIS K 7129). The temperature was 40 degrees by Celsius temperature scale (°C) and the relative humidity (RH) was 90%. The moisture was passed from a side that was to be set at the outer side of the pouched part to another side that was to be set at the inner side of the pouched part. For each exothermic composition, two samples were examined.

### (4) Preparations of Sheet-like Face Packs

Onto a side of a commercially available, air-impermeable polyethylene film (from Minacel; thickness: 40 *µ* m), an emulsion adhesive comprising an acrylic copolymer (from Konishi; No. CE310H) was entirely applied by using a rotary screen print (from Kaminoyama-Kikoh). By this adhesive, a polyester (100%), spun lace, non-woven fabric (from Komin non-woven fabric preparation company in Zhejiang, China; basis weight: 60 g/m²) was mounted onto the air-impermeable polyethylene film. Thus, a composite sheet A was obtained. Then, by using a coater the composition for gel (see Table 2) that had been prepared was applied onto the non-woven fabric of the composite sheet A so that the basis weight of the composition would come to be 1,200 g/m². The surface of the applied composition for gel was covered with a polypropylene emboss film (from Shin-Kansai film; thickness: 30 *µ* m) as a releasable sheet. Thus, a composite sheet A having a gel layer was prepared.

Separately, onto a side of a porous polyethylene film (from Daiwagawa Polymer; thickness: 50 *µ* m), an emulsion adhesive comprising an acrylic copolymer (from Konishi; No. CE310H) was partly applied by using a rotary screen print (from Kaminoyama-Kikoh). By this adhesive, a polyester (100%), spun lace, non-woven fabric (from Komin nonwoven fabric preparation company in Zhejiang, China; basis weight: 50 g/m²) was mounted onto the porous polyethylene film. Thus, a composite sheet B was obtained.

The adhesive was applied in a width of 2 mm so that parts where there were no adhesive had a form of diamond having a length of each side of, e.g., 10 mm. By varying the size of the diamond, the moisture permeabilities were varied as shown in Table 1.

On four portions each having a form of quarter circle (see Figure 9; the portions were traced with broken lines) of the air-impermeable polyethylene film of the composite sheet A having a gel layer, an exothermic composition (10 g/portion) was laid so that the thickness would be even (about 1.0 mm).

Next, the surfaces of the exothermic composition were covered with the composite sheet B wherein the porous polyethylene film faced the exothermic composition. The peripheries having a width of about 15 mm and the orthogonal two axial parts having a width of about 12 mm (see Figure 9; with the proviso that the actually-prepared face pack has a form of ellipse) were heat-sealed.

Thus, six kinds of sheet-like face packs each having a form of ellipse (major axis: 25 cm; minor axis: 22 cm) were prepared.

Each of the sheet-like face packs was put into a bag that had been made of a composite of aluminum foil and a polymer film.

### (5) Determinations of Properties of Increases and Maintenances of Temperatures of Sheet-like Face Packs

Based on the method that is provided in JIS S 4100, the temperatures of the gel layers of the sheet-like face packs were determined after the face packs had been taken out from the bags. For each exothermic composition, two samples were examined. Table 1 shows the results.

### Example 2: Preparation of Kit (I) for Face Pack

### (1) Preparation of Exothermic Structure (I)

The composite sheets A and B that had been prepared in Example 1 were used.

On four portions each having a form of quarter circle (see Figure 9; the portions were traced with broken lines) of the air-impermeable polyethylene film of the composite sheet A, an exothermic composition (10 g/portion) was laid so that the thickness would be even (about 1.0 mm).

Next, the surfaces of the exothermic composition were covered with the composite sheet B wherein the porous polyethylene film faced the exothermic composition. The peripheries having a width of about 15 mm and the orthogonal two axial parts having a width of about 12 mm (see Figure 9; with the proviso that the actually-prepared exothermic structure has a form of ellipse) were heat-sealed.

Thus, an exothermic structure (I) having a form of ellipse (major axis: 25 cm; minor axis: 22 cm) was prepared.

The exothermic structure (I) was put into a bag that had been made of a composite of aluminum foil and a polymer film.

### (2) Preparation of Sheet-like Gel

The composition for gel having the formula shown in Table 2 was prepared according to the method of Example 1. By using a coater the composition for gel was applied onto a polypropylene emboss film (from Shin-Kansai film; thickness: 30 *µ* m) so that the basis weight of the composition would come to be 1,200 g/m². The surface of the applied composition was covered with the same polypropylene emboss film.

After the gel had hardened, the sheet-like gel that was covered with two polypropylene emboss films was put into a bag that was made of a composite sheet of a KOP film and a PE film.

### (3) Use of Kit (I) for Face Pack

The exothermic structure (I) was taken out from the bag. The sheet-like gel that was covered with two polypropylene emboss films was taken out from the bag and then one polypropylene emboss film was peeled off. On the substrate layer for the gel, i.e., the non-woven fabric, the sheet-like gel having one polypropylene emboss film was put so that the gel faced the substrate layer. The other polypropylene emboss film was also peeled off and the gel of the thus-prepared face pack was touched to a face.

### Example 3: Preparation of Kit (II) for Face Pack

### (1) Preparation of Exothermic Structure (II)

A commercially available, air-impermeable polyethylene film (from Minacel; thickness: 40 *µ* m) and the composite sheet B that had been prepared in Example 1 were used.

On four portions each having a form of quarter circle (see Figure 9; the portions were traced with broken lines) of the air-impermeable polyethylene film, an exothermic composition (10 g/portion) was laid so that the thickness would be even (about 1.0 mm).

Next, the surfaces of the exothermic composition were covered with the composite sheet B wherein the porous polyethylene film faced the exothermic composition. The peripheries having a width of about 15 mm and the orthogonal two axial parts having a width of about 12 mm (see Figure 9; with the proviso that the actually-prepared exothermic structure (II) has a form of ellipse) were heat-sealed.

Thus, an exothermic structure (II) having a form of ellipse (major axis: 25 cm; minor axis: 22 cm) was prepared.

The exothermic structure (II) was put into a bag that had been made of a composite of aluminum foil and a polymer film.

### (2) Preparation of Gel

A composition for gel had the same formula as that shown in Table 2 except that dried alminium hydroxide gel and magnesium metasilicate aluminate were not contained. The composition for gel was prepared by a manner similar to that disclosed in Example 1.

The prepared gel was put into a tube.

### (3) Use of Kit (II) for Face Pack

The exothermic structure (II) was taken out from the bag. Onto the air-impermeable polyethylene film, the gel that had been squeezed from the tube was applied with fingers so that the thickness of the gel would come to be about 1 mm. Quickly, the gel of the thus-prepared face pack was touched to a face.

**Table 1 (Part 1)**

| | | Exothermic composition A | | Exothermic composition B | | Exothermic composition C | |
|---|---|---|---|---|---|---|---|
| Formulas of exothermic compostions | Iron powder | 49.5 | | 4 9.0 | | 48.5 | |
| | Active carbon | 8.0 | | 7.0 | | 6.0 | |
| | Wood powder | 9.0 | | 8.0 | | 9.5 | |
| | Carboxymethyl cellulose | 2.0 | | 2.0 | | 2.0 | |
| | Acrylic acid starch | 1.0 | | 2.0 | | 2.0 | |
| (% by weight) | Sodium chloride | 2.0 | | 2.0 | | 2.0 | |
| | Ordinary water | 28.5 | | 30.0 | | 30.0 | |
| | Total | 100.0 | | 100.0 | | 100.0 | |
| Results of experiments | Time necessary to reach 39°C | 9 minutes 30 seconds | 9 minutes 20 seconds | 8 minutes 30 seconds | 8 minutes | 7 minutes 40 seconds | 7 minutes 20 seconds |
| | Time maintained at a temperature of 39°C or higher | 2 hours 45 minutes | 2 hours 50 minutes | 2 hours 20 minutes | 2 hours 30 minutes | 2 hours 10 minutes | 2 hours |
| | The highest temperature (°C) | 44.6 | 43.2 | 44.3 | 43.8 | 44.7 | 43.5 |
| | Moisture permeability (g/m²·24hours) | 345 | 340 | 367 | 358 | 417 | 435 |

**Table 1 (Part 2)**

| | | Exothermic composition D | | Exothermic composition E | | Exothermic composition F | |
|---|---|---|---|---|---|---|---|
| Formulas of exothermic compositions | Iron powder | 48.0 | | 47.5 | | 47.0 | |
| | Active carbon | 4.0 | | 2.0 | | 2.0 | |
| | Wood powder | 9.5 | | 10.0 | | 10.0 | |
| | Carboxymethyl cellulose | 3.0 | | 3.0 | | 3.0 | |
| | Acrylic acid starch | 2.0 | | 2.0 | | 2.0 | |
| (% by weight) | Sodium chloride | 2.0 | | 2.0 | | 2.0 | |
| | Ordinary water | 31.5 | | 33.5 | | 34.0 | |
| | Total | 100.0 | | 100.0 | | 100.0 | |
| Results of experiments | Time necessary to reach 39°C | 6 minutes 30 seconds | 6 minutes 10 seconds | 6 minutes 40 seconds | 6 minutes | 6 minutes 10 seconds | 6 minutes |
| | Time maintained at a temperature of 39°C or higher | 1 hour 45 minutes | 1 hour 20 minutes | 55 minutes | 1 hour | 1 hour 10 minutes | 1 hour |
| | The highest temperature (°C) | 43.2 | 4.2.6 | 42.9 | 41.8 | 42.0 | 42.8 |
| | Moisture permeability (g/m²·24hours) | 529 | 614 | 689 | 778 | 804 | 835 |

## Claims

1. A sheet-like face pack comprising a pouched part having an air-permeable side and an air-impermeable side, a substrate layer for gel that is set on the outer surface of the air-impermeable side, a gel layer that is set on the substrate layer, and an exothermic composition that is put into the pouched part, wherein a moisture permeability of the air-permeable side is within the range of 300 to 2,300 g/m²·24hours by the Lyssy method (JIS K 7129, at 40 degrees by Celsius temperature scale and relative humidity of 90%), and wherein the temperature of the gel layer reaches 38 degrees by Celsius temperature scale (°C) within 10 minutes after the exothermic composition starts to contact oxygen and is then maintained between 38 °C and 45 °C for 20 minutes or more, wherein the temperature of the gel layer is determined according to the method for determining a temperature based on JIS S 4100.

2. The sheet-like face pack according to claim 1, wherein the substrate layer for gel is made of a non-woven fabric, a woven fabric, paper, or knit.

3. The sheet-like face pack according to claim 1 or 2, wherein the air-permeable side is a composite that comprises an outer layer of a material for retaining moisture and an inner layer that is made of an air-permeable polymer sheet wherein the outer layer partly adheres to the inner layer, and the air-impermeable side is made of an air-impermeable polymer sheet.

4. The sheet-like face pack according to any one of claims 1 to 3, wherein there are two or more pouched parts into which the exothermic composition is put.

5. The sheet-like face pack according to any one of claims 1 to 4, wherein the exothermic composition comprises, based on the total weight of the exothermic composition, 35.0 to 55.0% by weight of a metallic powder, 1.0 to 10.0% by weight of a reaction auxiliary, 1.0 to 10.0% by weight of a carbon powder, and 20.0 to 40.0% by weight of water.

6. The sheet-like face pack according to any one of claims 1 to 5, wherein the gel layer has been prepared from a composition comprising, based on the total weight of the composition, 5 to 15% by weight of a water-soluble polymeric compound, 0.05 to 1.00% by weight of a cross linking agent, 10 to 30% by weight of a polyhydric alcohol, and 40 to 80% by weight of water.

7. The sheet-like face pack according to claim 6, wherein the composition further comprises, based on the total weight of the composition, 0.01 to 10.00% by weight of a component that is effective to make or maintain skin healthy.

8. The sheet-like face pack according to claim 7, wherein the component is at least one member selected from the group consisting of a placenta extract, collagen, hyaluronic acid, coenzyme Q10, amino acids, vitamins, stearic acid, olive oil, carrot oil, essential oils, vegetable extracts, seaweed extracts, and fruit extracts.

9. A kit for a face pack comprising an exothermic structure that comprises a pouched part having an air-permeable side and an air-impermeable side, a substrate layer for gel that is set on the outer surface of the air-impermeable side, and an exothermic composition that is put into the pouched part, and gel that is separate from the exothermic structure, wherein a moisture permeability of the air-permeable side is within the range of 300 to 2,300 g/m². 24hours by the Lyssy method (JIS K 7129, at 40 degrees by Celsius temperature scale and relative humidity of 90%), and wherein within 10 minutes after preparing a gel layer by using the gel on the outer surface of the substrate layer, the temperature of the gel layer reaches 38 degrees by Celsius temperature scale (°C) and is then maintained between 38 °C and 45 °C for 20 minutes or more, wherein the temperature of the gel layer is determined according to the method for determining a temperature based on JIS S 4100.

10. The kit for a face pack according to claim 9, wherein the exothermic structure is put into an air-impermeable bag and wherein the gel has a sheet-like form and is put into a waterproof bag.

11. The kit for a face pack according to claim 10, wherein the gel has been prepared from a composition comprising, based on the total weight of the composition, 5 to 15% by weight of a water-soluble polymeric compound, 0.05 to 1.00% by weight of a cross linking agent, 10 to 30% by weight of a polyhydric alcohol, and 40 to 80% by weight of water.

12. A kit for a face pack comprising an exothermic structure that comprises a pouched part having an air-permeable side and an air-impermeable side and an exothermic composition that is put into the pouched part, and gel that is separate from the exothermic structure, wherein a moisture permeability of the air-permeable side is within the range of 300 to 2,300 g/m²· 24hours by the Lyssy method (JIS K 7129, at 40 degrees by Celsius temperature scale (°C) and relative humidity (RH) of 90%), and wherein within 10 minutes after preparing a gel layer by using the gel on the outer surface of the air-impermeable side, the temperature of the gel layer reaches 38 degrees by Celsius temperature scale (°C) and is then maintained between 38 °C and 45 °C for 20 minutes or more, wherein the temperature of the gel layer is determined according to the method for determining a temperature based on JIS S 4100.

13. The kit for a face pack according to claim 9 or 12, wherein the exothermic structure is put into an air-impermeable bag and wherein the gel is put into a tube or a waterproof pouch.

14. The kit for a face pack according to claim 13, wherein the gel has been prepared from a composition comprising, based on the total weight of the composition, 5 to 15% by weight of a water-soluble polymeric compound, 10 to 30% by weight of a polyhydric alcohol, and 40 to 80% by weight of water.
